# EUROPEAN PATENT SPECIFICATION

(11) **EP 2 822 508 B1**
(45) Date of publication and mention of the grant of the patent: **02.03.2016**
(21) Application number: 13715785.5
(22) Date of filing: 06.03.2013
(51) Int. Cl.: A61F 2/30, A61F 2/38

(54) **FEMORAL AUGMENTS FOR USE WITH KNEE JOINT PROSTHESIS**
FEMURVERSTÄRKUNGEN ZUM GEBRAUCH MIT EINER KNIEGELENKPROTHESE
COMPLÉMENTS FÉMORAUX DESTINÉS À ÊTRE UTILISÉS AVEC UNE PROTHÈSE D'UNE ARTICULATION DU GENOU

(30) Priority: 09.03.2012 US 201213416857
(43) Date of publication of application: 14.01.2015
(73) Proprietor: Zimmer, Inc., Warsaw, Indiana 46580 (US)
(72) Inventor: HOEMAN, Timothy A., Morris Plains, NJ 07950 (US)
(74) Representative: Mays, Julie
(86) International application number: PCT/US2013/029251
(87) International publication number: WO 2013/134333

(56) References cited:
- EP-A1- 1 913 902
- EP-A1- 2 130 518
- DE-A1-102010 044 571
- US-A- 4 822 366

## Description

### CLAIM OF PRIORITY

Benefit of priority is hereby claimed to U.S. Patent Application Serial Number 13/416,857, entitled "FEMORAL AUGMENTS FOR USE WITH KNEE JOINT PROSTHESIS," filed on March 9, 2012.

### BACKGROUND

### 1. Technical Field.

The present disclosure relates to orthopaedic prostheses, and more particularly, to modular support structures for use with a knee prosthesis.

### 2. Description of the Related Art.

Orthopaedic prostheses are commonly utilized to repair and/or replace damaged bone and tissue in the human body. For example, a knee prosthesis may be used to restore natural knee function by repairing damaged or diseased articular surfaces of the femur and/or tibia. Knee prostheses may include a femoral component implanted on the distal end of the femur, which articulates with a tibial component implanted on the proximal end of a tibia to replicate the function of a healthy natural knee.

One goal of knee replacement procedures is to reproduce or enhance the kinematics of the natural knee using the associated prosthetic components. More generally, such procedures seek to achieve kinematic characteristics that promote favorable patient outcomes such as minimized pain, proper joint function through a wide range of motion, and the longest possible prosthesis service life.

However, in some cases the proximal tibia and/or distal femur may have severe degeneration, trauma, or other pathologies which necessitates resection of more bone than can be compensated for by traditional femoral and tibial components. For example, in the proximal tibia poor quality bone stock may exist around the medullary canal in the diaphyseal and/or metaphyseal region. In such cases, a surgeon may opt for an augment having a generally cone shaped outer profile corresponding to the generally cone shaped bone defect typically encountered around the medullary canal. Exemplary tibial cone augments are disclosed in U.S. Patent Application Publication No. 2007/0088443, filed November 15, 2006 and entitled PROSTHETIC IMPLANT SUPPORT STRUCTURE, and in U.S. Patent Application Serial No. 2011/0009974, filed September 20, 2010 and entitled TIBIAL AUGMENTS FOR USE WITH KNEE JOINT PROSTHESES, METHOD OF IMPLANTING THE TIBIAL AUGMENT, AND ASSOCIATED TOOLS.

Similarly in the distal femur poor quality bone stock may also exist around the femoral medullary canal. A distal femoral augment having a tapered outer configuration may be implanted to fill the void formed by removal of this poor quality bone stock, such as the distal femoral augments disclosed in U.S. Patent Application Publication No. 2011/0112651, filed January 14, 2011 and entitled FEMORAL AUGMENTS FOR USE WITH KNEE JOINT PROSTHESIS. EP1913902 discloses a modular femoral joint prosthesis which includes a femoral component having a post extending therefrom, a femoral stem, and a femoral sleeve configured to be coupled to the post of the femoral assembly and to the femoral stem. EP2130518 discloses a monolithic foam sleeve comprising titanium or titanium alloy having a proximal end, a distal end, an interior wall that defines an interior channel and extends from the proximal end to the distal end wherein the outer surface tapers such that said sleeve is widest at the distal end and most narrow at the proximal end. US4822366 discloses a modular knee prosthesis comprising a femoral component, and a tibial component that includes a separate tibial insert that is configured to be supported by the tibial component platform.

### SUMMARY

The invention is defined in claim 1. The present disclosure provides a modular, multi-piece implant support structure system which promotes stable, long-term fixation of a distal femoral implant in femurs having damage and/or disease with varying degrees of severity. The modular support structure system facilitates selective augmentation of metaphyseal and/or diaphyseal bone surrounding the femoral medullary canal. In one exemplary embodiment, the system utilizes augment components which can be assembled into a large augment assembly, and can be used independently of one other.

Described is a femoral augment system comprising: a first augment comprising a proximal body, a medial leg extending distally from the proximal body, and a lateral leg extending distally from the proximal body and spaced from the medial leg to define a recess between the medial and lateral legs, the proximal body defining a first augment mating surface disposed opposite the medial and lateral legs; and a second augment with tapered outer surface extending longitudinally from a proximal end surface to a distal end surface, the distal end surface defining a second augment mating surface, the first augment mating surface substantially congruent to the second augment mating surface.

The present disclosure provides a femoral augment system comprising: a first augment comprising a proximal body, a medial leg extending distally from the proximal body, and a lateral leg extending distally from the proximal body and spaced from the medial leg to define a recess between the medial and lateral legs, the proximal body defining a tapered outer surface; and a second augment with tapered outer surface extending longitudinally from a proximal end surface to a distal end surface, the tapered outer surfaces of the first and second augments complementary to one another, such that the first and second augments are assemblable to one another to form to form a substantially continuous tapered surface.

### BRIEF DESCRIPTION OF THE DRAWINGS

The above mentioned and other features and advantages of this disclosure, and the manner of attaining them, will become more apparent and the invention itself will be better understood by reference to the following description of embodiments of the invention taken in conjunction with the accompanying drawings, wherein:
FIG. 1 is a perspective view of modular augment components made in accordance with the present disclosure;
Fig. 2 is a distal, perspective, exploded view of the modular augments shown in Fig. 1, together with a distal femoral prosthesis and a distal femur resected to receive the prosthesis/augment combination;
Fig. 3 is an anterior, elevation view of the modular prosthesis components shown in Fig. 1, assembled to the femoral prosthesis shown in Fig. 2; and
FIG. 4 is an anterior, elevation, section view of the assembled augment/prosthesis combination shown in Fig. 3.

Corresponding reference characters indicate corresponding parts throughout the several views. The exemplifications set out herein illustrate exemplary embodiments of the present invention, and such exemplifications are not to be construed as limiting the scope of the invention in any manner.

### DETAILED DESCRIPTION

The present disclosure provides an implant augment system that allows a surgeon to selectively choose varying levels of augmentation by using one or more augment components from a set of augment components. The support structure components may be made of a porous bone ingrowth material that provides a scaffold for bone ingrowth on multiple surfaces. These surfaces present large, three-dimensional areas of bone ingrowth material to the surrounding healthy bone for secure and stable long term fixation of the support structure to the distal femur. An articular femoral component may be attached to the support structure.

Support structure in accordance with the present disclosure may be formed from a single piece of highly porous biomaterial. A highly porous biomaterial is useful as a bone substitute and as cell and tissue receptive material. A highly porous biomaterial may have a porosity as low as 55%, 65%, or 75% or as high as 80%, 85%, or 90%. An example of such a material is produced using Trabecular Metal™ Technology generally available from Zimmer, Inc., of Warsaw, Indiana. Trabecular Metal™ is a trademark of Zimmer, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 to Kaplan and 6,103,149 to Stankiewicz. In addition to tantalum, other metals such as niobium, or alloys of tantalum and niobium with one another or with other metals may also be used.

Generally, the porous tantalum structure includes a large plurality of ligaments defining open spaces therebetween, with each ligament generally including a carbon core covered by a thin film of metal such as tantalum, for example. The open spaces between the ligaments form a matrix of continuous channels having no dead ends, such that growth of cancellous bone through the porous tantalum structure is uninhibited. The porous tantalum may include up to 75%, 85%, or more void space therein. Thus, porous tantalum is a lightweight, strong porous structure which is substantially uniform and consistent in composition, and closely resembles the structure of natural cancellous bone, thereby providing a matrix into which cancellous bone may grow to provide fixation of the support structure to the patient's bone.

The porous tantalum structure may be made in a variety of densities in order to selectively tailor the structure for particular applications. In particular, as discussed in U.S. Patent No. 5,282,861, the porous tantalum may be fabricated to virtually any desired porosity and pore size, and can thus be matched with the surrounding natural bone in order to provide an improved matrix for bone ingrowth and mineralization.

Porous-material augments in accordance with the present disclosure may be utilized in combination with materials which stimulate bone ingrowth into the porous tantalum structure. For example, the augments themselves may be coated or infused with such materials, or the bone-ingrowth materials may be used in lieu of bone cement (as described in detail below). Exemplary bone stimulus materials are described in U.S. Provisional Patent Application Serial No. 61/584,460 (Attorney Docket No. ZIM0908), filed January 9, 2012, entitled COMPOSITE DEVICE THAT COMBINES POROUS METAL AND BONE STIMULI and commonly assigned with the present application.

As used herein, "proximal" refers to a direction generally toward the torso of a patient, and "distal" refers to the opposite direction of proximal, i.e., away from the torso of a patient. "Anterior" refers to a direction generally toward the front of a patient or knee, and "posterior" refers to the opposite direction of anterior, i.e. toward the back of the patient or knee. In the context of a prosthesis alone, such directions correspond to the orientation of the prosthesis after implantation, such that a proximal portion of the prosthesis is that portion which will ordinarily be closest to the torso of the patient, the anterior portion closest to the front of the patient's knee, etc.

Similarly, knee prostheses and augments in accordance with the present disclosure may be referred to in the context of a prosthesis coordinate system including three mutually perpendicular reference planes, referred to herein as the transverse, coronal and sagittal planes of the knee prosthesis. Upon implantation and with a patient in a standing position, a transverse plane of the knee prosthesis is generally parallel to an anatomic transverse plane, i.e., the transverse plane is inclusive of imaginary vectors extending along medial/lateral and anterior/posterior directions. Coronal and sagittal planes of the knee prosthesis are also generally parallel to the coronal and sagittal anatomic planes in a similar fashion. Thus, a coronal plane of the prosthesis is inclusive of vectors extending along proximal/distal and medial/lateral directions, and a sagittal plane is inclusive of vectors extending along anterior/posterior and proximal/distal directions. As with anatomic planes, the sagittal, coronal and transverse planes of a knee prosthesis are mutually perpendicular to one another. For purposes of the present disclosure, reference to sagittal, coronal and transverse planes is with respect to a knee prosthesis unless otherwise specified.

In an exemplary embodiment, a system of different stock sizes of augments are made available, as discussed more fully below, with different sizes being used for filling different sized defects in different sized femurs. Further, each of the different sizes of femoral augments may be available in a variety of heights (measured along a distal/proximal direction). By providing femoral augments of different sizes, with different heights available for each of the sizes, the optimal size and height augment can be selected so that only a minimal amount of healthy bone needs to be removed, thereby promoting the early onset of bony in-growth. Additionally, the configuration of the femoral augments, as well as the availability of various sizes and heights, allows for the preservation of a significant amount of peripheral bone, whereby such bone can later grow into and infiltrate the porous material of the augment and/or the femoral component of the implant to restore the bony platform upon which the augment and the implant reside.

The embodiments shown and described herein illustrate components associated with a left knee prosthesis. Right and left knee prosthesis configurations are mirror images of one another about a sagittal plane. Thus, it will be appreciated that all aspects of the prosthesis described herein are equally applicable to left- or right-knee prosthesis configurations.

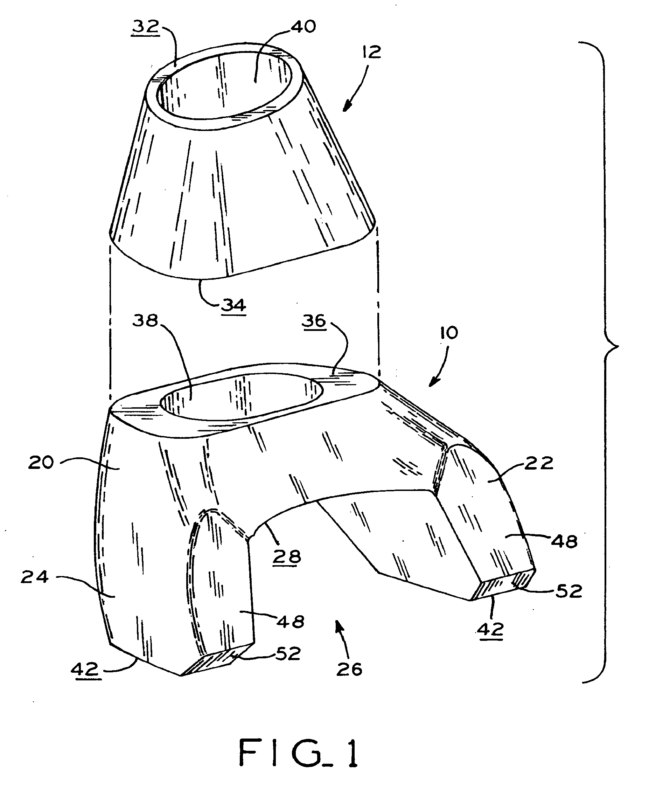

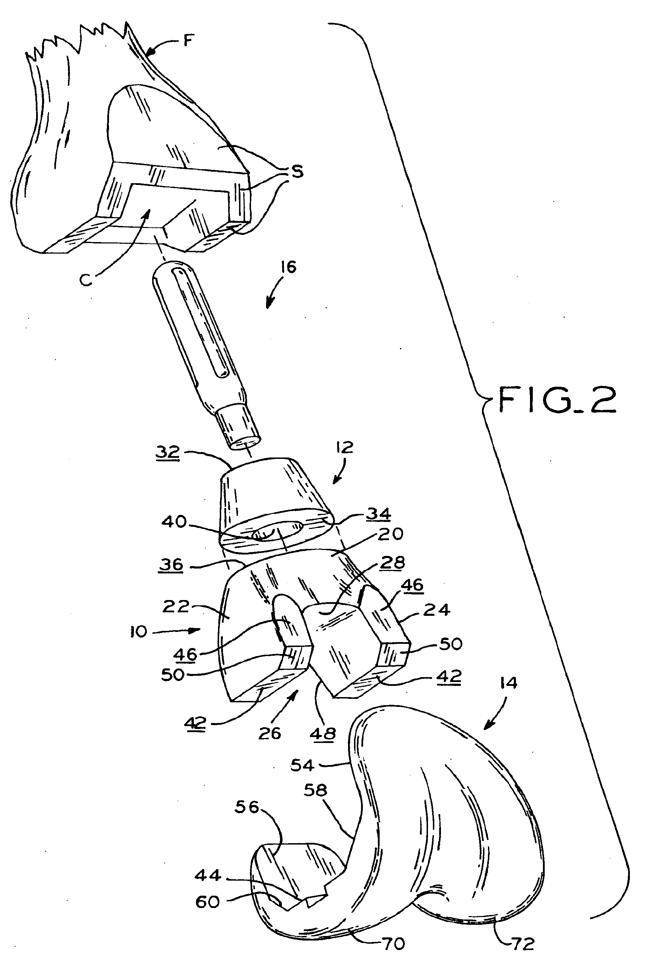

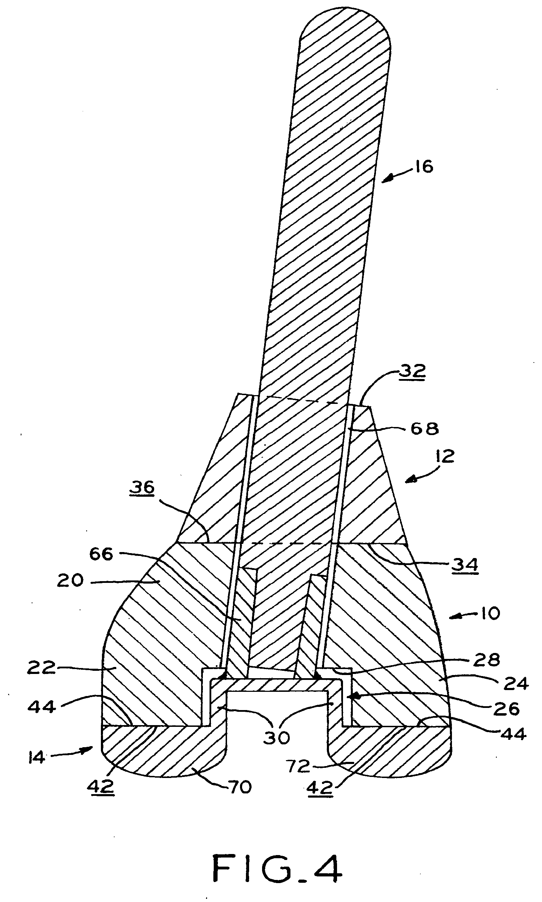

Turning now to Fig. 1, a pair of modularly attachable femoral augments are illustrated which are sized and shaped to be used in a distal femur in the vicinity of the intramedullary canal. Metaphyseal augment 10 includes a proximal body 20 with medial leg 22 and lateral leg 24 extending distally from distal surface 28 of proximal body 20. Recess 26 formed between medial and lateral legs 22, 24 has a generally U-shaped coronal profile, and is sized to receive intercondylar walls 30 of femoral component 14, as shown in Fig. 4 and described below. Diaphyseal augment 12 has a generally conical outer surface truncated at a proximal end thereof by proximal surface 32 and at a distal end thereof by distal surface 34. As described in detail below, distal surface 34 defines an outer surface periphery which is congruent with proximal surface 36 of metaphyseal augment 10 such that diaphyseal augment 12 is modularly attachable to metaphyseal augment 10. Thus, the total augmented region within cavity C of distal femur F (Fig. 2) can be selectively expanded by mating distal surface 34 of diaphyseal augment 12 to proximal surface 36 ofmetaphyseal augment 10.

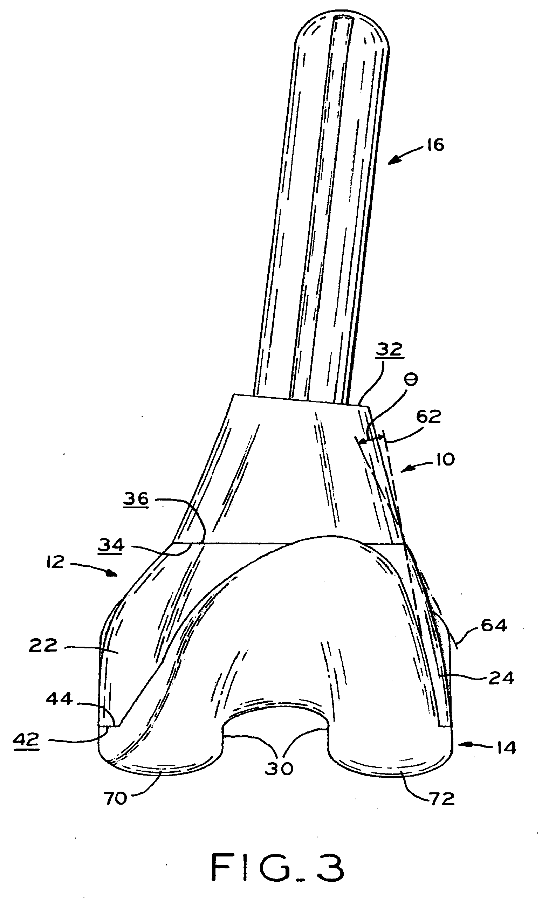

In an exemplary embodiment, metaphyseal and diaphyseal augments 10, 12 are each monolithically formed from a single piece of porous metal material (described above). Affixation of augments 10, 12 to one another, as described herein, forms a single integral distal femoral augment as shown in Figs. 3 and 4.

Turning to Fig. 2, an exploded view of a prosthesis system utilizing metaphyseal and diaphyseal augments 10, 12 is shown. Metaphyseal augment 10 may attach directly to femoral component 14, and diaphyseal augment 12 may attach to metaphyseal augment 10. Stem extension 16 attaches to femoral component 14, and passes through apertures 38, 40 of augments 10, 12 respectively. Augments 10, 12 and stem extension 16 are sized to be received within a prepared cavity C of the metaphyseal and diaphyseal regions of femur F, while femoral component 14 may be received upon correspondingly resected surfaces S of femur F.

Metaphyseal augment 10 affixes to the nonarticulating surfaces of femoral component 14. Femoral component 14 includes medial and lateral condyles 70, 72 which define convex outer surfaces adapted to articulate with a tibial articular surface. Opposite the convex articular surfaces of medial and lateral condyles 70, 72 are nonarticular surfaces of femoral component 14. For example, as best seen in Fig. 2, metaphyseal augment 10 includes distal surfaces 42 on each of medial and lateral legs 22, 24, which are adapted to abut or nearly abut the corresponding distal facet 44 of femoral component 14 (Fig. 4). Similarly, anterior surfaces 46, posterior surfaces 48 (Fig. 1), anterior chamfers 50 and posterior chamfers 52 (Fig. 1) are sized and positioned to abut or nearly abut anterior facet 54, posterior facet 56, anterior chamfer facet 58 and posterior chamfer facet 60, respectively. In an exemplary embodiment, metaphyseal augment 10 is spaced slightly from femoral component 14 by between about 5 and 10 mm upon affixation thereto, with a layer of bone cement occupying this 5 to 10 mm gap to effect fixation therebetween.

Recess 26 between medial and lateral legs 22, 24 may be sized to accommodate intercondylar walls 30 of femoral component 14, as noted above. As best seen in Fig. 4, intercondylar walls 30 extend in a generally proximal direction from distal facet 44 to define a total proximal/distal extent that is greater than the corresponding proximal/distal extent of intercondylar walls 30, thereby providing clearance therebetween when metaphyseal augment 10 is affixed to femoral component 14 as shown. In some prostheses, intercondylar walls 30 may be omitted from femoral component 14, or may be reduced in height. Recess 26 may be similarly reduced in proximal/distal extent in order to accommodate intercondylar walls 30 of any configuration.

An exemplary metaphyseal augment 10 is disclosed in U.S. Patent No. 7,892,288, filed March 5, 2004 and entitled "FEMORAL AUGMENTS FOR USE WITH KNEE JOINT PROSTHESIS", and in U.S. Patent Application Publication No. 2011/0112651, filed January 14, 2011 and entitled "FEMORAL AUGMENTS FOR USE WITH KNEE JOINT PROSTHESIS". Exemplary femoral components 14 include the LCCK and Rotating Hinge Knee femoral components available from Zimmer, Inc. of Warsaw, Indiana. LCCK femoral components are shown in "Zimmer® NexGen® LCCK, Surgical Technique for use with LCCK 4-in-1 Instrumentation", and the Rotating Hinge Knee femoral components are shown in " Zimmer® NexGen® Rotating Hinge Knee Primary/Revision, Surgical Technique", each of which is filed on even date herewith in an Information Disclosure Statement. However, it is contemplated that metaphyseal augment 10 may be used with a wide variety of femoral component designs, including femoral components made by various manufacturers.

Diaphyseal augment 12 assembles to metaphyseal augment 10 by mating or otherwise associating distal surface 34 of diaphyseal augment 12 to proximal surface 36 of metaphyseal augment 10, as noted above. The outer periphery of distal surface 34 is substantially congruent to the corresponding outer periphery of proximal surface 36, such that no portion or only a very small portion of distal surface 34 or proximal surface 36 is visible when surfaces 34, 36 are properly mated. Stated another way, the outer periphery of distal surface 34 is substantially identical to the outer periphery of proximal surface 36, such that the two outer peripheries of surfaces 34, 36 are congruent when properly aligned. Advantageously, the congruency of the outer peripheries of surfaces 34, 36 facilitates assembly of metaphyseal and diaphyseal augments 10, 12 into a single, unitary distal femoral augment. That is to say, when distal surface 34 of diaphyseal augment 12 is properly aligned and mated with proximal surface 36 of metaphyseal augment 10, no sharp edges or abrupt surface transitions exist at the junction between surfaces 34, 36.

For example, proximal body 20 of metaphyseal augment 10 may define a proximally-converging, tapered conical outer surface near proximal surface 36. This conical outer surface is complimentary to a correspondingly tapered outer surface of diaphyseal augment 12 near distal surface 34. Thus, when metaphyseal and diaphyseal augments 10, 12 are assembled such that the outer peripheries of distal and proximal surfaces 34, 36 are congruently aligned, the tapered outer surfaces of augments 10, 12 blend into a substantially smooth, uninterrupted surface spanning the junction between distal and proximal surfaces 34, 36.

Referring to Fig. 3 the uninterrupted surface of the integral distal femoral augment formed by the affixed augments 10, 12 may be quantified by observing the relationship between tangent lines 62, 64. Tangent line 62 is drawn tangent to the illustrated coronal profile of proximal body 20 of metaphyseal augment 10 near proximal surface 36. Tangent line 64 is drawn tangent to the coronal profile of diaphyseal augment 12 near distal surface 34. Because the outer surfaces of augments 10 and 12 are complimentary when augments 10, 12 are mated to form an integral distal femoral augment, tangent lines 62, 64 are nearly parallel. Exactly parallel tangent lines 62, 64 at taken at the junction between proximal and distal surfaces 36, 34 implies a perfectly smooth, uninterrupted transition from the outer tapered periphery of proximal body 20 of metaphyseal augment 10 at proximal surface 36 to the correspondingly tapered outer surface of diaphyseal augment 12 at distal surface 34. It is contemplated that such tangent lines 62, 64 may be parallel in one exemplary embodiment, or may deviate slightly from one another as shown in Fig. 3 to form angle θ therebetween. Angle θ may range from as little as zero degrees to as much as 5 or 10 degrees, or may be any value within any range defined by the foregoing values. Moreover, it is contemplated that angle θ may vary depending on where such an angle is taken around the peripheries of surfaces 34, 36.

As best seen in Fig. 4, the inner peripheries of proximal and distal surfaces 36, 34 of metaphyseal and diaphyseal augments 10, 12, respectively (i.e., the peripheries of apertures 38, 40) may also be congruent in a similar fashion to the outer peripheries as described above. This congruence creates a continuous aperture formed of apertures 38, 40 when augments 10, 12 are assembled to one another. As illustrated, stem extension 16 passes through this continuous aperture and extends proximally away from proximal surface 32 of diaphyseal augment 12. This exposed, proximally extending portion of stem 16 is positioned and oriented to extend into the intramedullary canal of femur F (Fig. 2) upon implantation in order to more securely seat the femoral component 14 within femur F. At its distal end, stem extension 16 is attached to stem base 66 of femoral component 14.

As illustrated in Fig. 4, a gap or space 68 is formed between the inwardly facing walls of apertures 38, 40 and the outer surface of stem extension 16. Gap 68 gives rise to an annular void between apertures 38, 40 and stem extension 16, and this annular void extends along the entire axial extent of apertures 38, 40 when augments 10, 12 are assembled to one another as described herein. Gap 68 may be filled with a filler material, such as bone cement or morselized bone, or maybe left unfilled.

As described above, diaphyseal augment 12 may be sized and shaped to combine with metaphyseal augment 10 to create a unitary, modular distal femoral augment. However, diaphyseal augment 12 may also be useable as a metaphyseal augment in the proximal end of the human tibia. An exemplary metaphyseal tibial augment which is also useable as diaphyseal augment 12 is disclosed in U.S. Patent Application Publication No. 2007/0088443, filed November 15, 2006 and entitled "PROSTHETIC IMPLANT SUPPORT STRUCTURE". Thus, where diaphyseal augment 12 is provided as part of a kit or set of augments for knee arthroplasty, a separate augment or set of augments for the metaphyseal region of the proximal tibia may not need to be provided separately.

The tapered outer surfaces of proximal body 20 of metaphyseal augment 10 and diaphyseal augment 12 are each tapered inwardly in a proximal direction. These tapered surfaces are shaped to correspond with the inward taper of the metaphyseal and diaphyseal regions of the distal portion of femur F (Fig. 2). In some cases, metaphyseal and/or diaphyseal augments 10, 12 may be symmetric about sagittal and/or coronal planes, thereby rendering augments 10, 12 suitable for use with correspondingly symmetric bone defects in the distal femur. In other instances, augments 10 and/or 12 may be asymmetric, rendering augments 10, 12 appropriate to other, asymmetric bone defects. Moreover, it is contemplated that metaphyseal and diaphyseal augments 10, 12 may be symmetric or asymmetric about sagittal and/or coronal planes. In addition, diaphyseal augment 12 may be rotationally symmetric or asymmetric about the longitudinal axis of augment 12 (e.g., the axis of aperture 40). Where symmetric augments are employed, such augments may be used in the left or right knee interchangeably; where asymmetric components are employed, such augment components may be specifically adapted for use in a left or right knee.

It is further contemplated that a set or kit of metaphyseal and diaphyseal augments 10, 12 may be provided in varying sizes and/or geometric configurations. For example, each of metaphyseal and diaphyseal augments 10, 12 may be provided in a variety of augment heights. That is to say, a set of metaphyseal and/or diaphyseal augments 10, 12 may be provided in which each augment in the set has a different proximal distal extent as compared to the other comparable augments while retaining similar geometry (e.g., taper configurations, configurations of apertures 38, 40, medial/lateral extent, etc.). Similarly, a set of metaphyseal and diaphyseal augments 10, 12 of differing augment sizes may be provided, in which each size augment corresponds to a unique size of femoral component 14. For example, LCCK and Rotating Hinge Knee femoral components (described above) available from Zimmer, Inc. of Warsaw, Indiana are available in sizes designated as B, C, D, E and F, with B being the smallest size and F being the largest size. Each of metaphyseal and diaphyseal augments 10, 12 may be provided in a unique augment size to correspond with each unique size of femoral component 14.

A method of utilizing augments made in accordance with the present disclosure will now be described. If the surgeon for a knee joint replacement surgical technique first makes a determination as to whether there is significant bone loss in the distal femur, and whether such bone loss may warrant the use of one or both of metaphyseal and diaphyseal femoral augments 10, 12. If only metaphyseal augment 10 is to be used, the surgeon estimates the proper size and height of femoral augment 10 selects the intended implanted position of the femoral augment. If both metaphyseal and diaphyseal femoral augments 10, 12 are to be used, the surgeon estimates the proper size and height of a congruently mating pair of femoral augments 10 and 12, and selects the intended implanted position of the combined, unitary femoral augment formed by joining augments 10, 12.

In the above-described exemplary set or kit of augments 10, 12, a wide variety of sizes and heights enable the surgeon to find an appropriate augment or mating pair of augments for filling a cavitary defect that will only require minor shaping of the defect, such as with a rasp or burr tool. Accordingly, only a minimal amount of healthy bone should need to be removed in order to prepare a cavity for receiving one of the femoral augments of the present invention.

Cavity C for receiving augments 10, 12 is prepared within the distal portion of femur F, as illustrated in Fig. 2. Depending on the extent and size of the cavitary defect, the surgeon can assess (e.g., through preoperative imagining or an intraoperative visual assessment) whether metaphyseal augment 10 would be sufficient to fill the void created by resection of the defect, or whether both metaphyseal and diaphyseal augments 10, 12 are necessary. Thus, preparation of cavity C should only involve slight shaping of the existing cavitary defect so that the existing cavitary defect better conforms to the shape of the femoral augment being implanted. Such shaping can be performed using, for example, a burr tool or a rasp. As can be seen in FIG. 2, cavity C does not extend to the peripheral portions of femur F. Accordingly, the peripheral portions of the bone, if healthy, are preserved. In use, one or both of femoral augments 10, 12 are implanted into cavity C. Femoral component 14, with stem extension 16 attached thereto, is subsequently implanted into the femur using any desired implantation technique.

Advantageously, healthy bone removal is minimized because the present femoral augments provide the surgeon with some flexibility with regard to the overall size of the implanted augment, as well as the implantation location and orientation of the augment. For example, each of the present femoral augments may be positioned in any one location chosen from a range of locations that are at different distances from a femoral component. Further, diaphyseal augment 12 may be included or excluded in the augment system, thereby allowing flexibility in the overall proximal/distal extent and overall volume of the final augmented region. Thus, with such versatility in implantation location, augment size and augment height being provided with the present invention, the surgeon should be able to select an augment size and height (both by selecting from among a range of sizes, and by selectively including or excluding diaphyseal augment 12), an implanted location, and an implanted orientation that only requires minimal rasping or use of a burr tool to prepare a cavity for receiving the selected femoral augment.

If diaphyseal augment 12 is selected for use within cavity C of femur F, augment 12 may be affixed to augment 10 with proximal and distal surfaces 36, 34 thereof congruently mated as described above. In an exemplary embodiment, affixation of augments 10, 12 to one another may be accomplished by placing a layer of bone cement between surfaces 34 and 36. Augments 10, 12 can be joined in this fashion either before or after implantation into cavity C of femur F. Alternatively, augments 10, 12 can be affixed to one another by any other suitable method, such as by fasteners.

Then, the femoral augments 10 and/or 12 are inserted into cavity C by any desired method. After properly seating the augment(s) 10, 12 within the cavity, cement is applied to the inner cavity surfaces of the augment and the inner surface of femoral component 14. Next, femoral component 14, with stem extension 16 attached thereto, is attached to augment(s) 10 and/or 12 and to the peripheral bone remaining around the cavity. Then, the remainder of the knee joint prosthesis is attached using any desired method, and the surgical procedure continues in the customary manner.

While the present system has been described as having exemplary designs, the present invention can be further modified within the scope of the claims. This application is therefore intended to cover such departures from the present disclosure as come within known or customary practice in the art to which this disclosure pertains and which fall within the limits of the appended claims.

## Claims

1. A femoral augment system comprising:
a first augment (10) comprising a proximal body (20), a medial leg (22) extending distally from said proximal body, and a lateral leg (24) extending distally from said proximal body and spaced from said medial leg to define a recess (26) between said medial and lateral legs, said proximal body defining a tapered outer surface; and
a second augment (12), **characterised in that** said second augment has a tapered outer surface extending longitudinally from a proximal end surface (32) to a distal end surface (34),
wherein said tapered outer surfaces of said first (10) and second (12) augments are complementary to one another, such that said first and second augments are assemblable to one another to form a substantially continuous tapered surface.

2. The femoral augment system of claim 1, wherein said first augment (10) comprises a first augment mating surface (36) disposed opposite said medial (22) and lateral (24) legs, said second augment (12) comprises a second augment mating surface (34) at said distal end surface, said femoral augment system in said assembled configuration when said first augment mating surface abuts said second augment mating surface.

3. The femoral augment system of claim 2, wherein said first augment mating surface (36) is substantially congruent to said second augment mating surface (34).

4. The femoral augment system of claim 2, wherein:
said tapered outer surface of said first augment (10) defines a first tangent line (62) drawn at said first augment mating surface (36) in a coronal plane,
said tapered outer surface of said second augment (12) defines a second tangent line (64) drawn at said second augment mating surface (34) in the coronal plane, and
said first tangent line defining an angle (θ) with said second tangent line of less than 10 degrees.

5. The femoral augment system of any one of claims 1-4, wherein said first augment (10) is sized and shaped to fit into a metaphyseal region of a distal femur (F).

6. The femoral augment system of any one of claims 1-4, wherein said second augment (12) is sized and shaped to fit into one of a diaphyseal region of a distal femur (F) and a metaphyseal region of a proximal tibia.

7. The femoral augment system of any one of claims 1-4, wherein at least one of said first augment (10) and said second augment (12) is a highly porous biomaterial.

## Patentansprüche

1. Femorales Augmentationssystem, umfassend:
ein erstes Augment (10), umfassend einen proximalen Körper (20), einen medialen Schenkel (22), der sich distal von dem proximalen Körper erstreckt und einen lateralen Schenkel (24), der sich distal von dem proximalen Körper erstreckt und von dem medialen Schenkel beabstandet ist, um eine Aussparung (26) zwischen den medialen und lateralen Schenkeln zu definieren, wobei der proximale Körper eine konische Außenfläche definiert; und
ein zweites Augment (12), **dadurch gekennzeichnet, dass** das zweite Augment eine konische Außenfläche aufweist, die sich längs von einer proximalen Stirnfläche (32) zu einer distalen Stirnfläche (34) erstreckt,
wobei die konischen Außenflächen der ersten (10) und zweiten (12) Augmente zueinander komplementär sind, sodass die ersten und zweiten Augmente zusammengesetzt werden können, um eine im Wesentlichen durchgehende konische Fläche zu bilden.

2. Femorales Augmentationssystem nach Anspruch 1, wobei das erste Augment (10) eine erste Augmentpassfläche (36) umfasst, die gegenüber von den medialen (22) und lateralen (24) Schenkeln angeordnet ist, wobei das zweite Augment (12) an der distalen Stirnfläche eine zweite Augmentpassfläche (34) umfasst, wobei sich das femorale Augmentationssystem in der zusammengesetzten Konfiguration befindet, wenn die erste Augmentpassfläche an der zweiten Augmentpassfläche anliegt.

3. Femorales Augmentationssystem nach Anspruch 2, wobei die erste Augmentpassfläche (36) im Wesentlichen zu der zweiten Augmentpassfläche (34) kongruent ist.

4. Femorales Augmentationssystem nach Anspruch 2, wobei:
die konische Außenfläche des ersten Augments (10) eine erste Berührungslinie (62) definiert, die an der ersten Augmentpassfläche (36) in einer Koronalebene gezogen wird,
die konische Außenfläche des zweiten Augments (12) eine zweite Berührungslinie (64) definiert, die an der zweiten Augmentpassfläche (34) in der Koronalebene gezogen wird und
die erste Berührungslinie mit der zweiten Berührungslinie einen Winkel (θ) von weniger als 10 Grad definiert.

5. Femorales Augmentationssystem nach einem der Ansprüche 1-4, wobei das erste Augment (10) bemessen und geformt ist, um in einen metaphysären Bereich einer distalen Femur (F) zu passen.

6. Femorales Augmentationssystem nach einem der Ansprüche 1-4, wobei das zweite Augment (12) bemessen und geformt ist, um in einen eines diaphysären Bereichs einer distalen Femur (F) und eines metaphysären Bereichs einer proximalen Tibia zu passen.

7. Femorales Augmentationssystem nach einem der Ansprüche 1-4, wobei mindestens eines des ersten Augments (10) und des zweiten Augments (12) ein hochporöses Biomaterial ist.

## Revendications

1. Un système de tige fémorale, comprenant :
une première tige (10) comprenant un corps proximal (20), une jambe interne (22) s'étendant dans un plan distal depuis ledit corps proximal, et une jambe latérale (24), s'étendant dans un plan distal depuis ledit corps proximal et espacée de ladite jambe interne en définissant un évidement (26) entre lesdites jambes interne et latérale, ledit corps proximal définissant une surface extérieure conique ; et
une deuxième tige (12), **caractérisée en ce que** ladite deuxième tige possède une surface extérieure conique s'étendant longitudinalement d'une surface d'extrémité proximale (32) à une surface d'extrémité distale (34),
lesdites surfaces extérieures coniques desdites première (10) et deuxième (12) tiges étant complémentaires l'une de l'autre, de sorte que lesdites première et deuxième tiges peuvent être assemblées l'une avec l'autre pour former une surface conique substantiellement continue.

2. Le système de tige fémorale selon la revendication 1, ladite première tige (10) comprenant une surface de raccordement (36) de la première tige, disposée en face desdites jambes interne (22) et latérale (24), ladite deuxième tige (12) comprenant une surface de raccordement (34) de la deuxième tige sur ladite surface d'extrémité distale, ledit système de tige fémorale dans ladite configuration assemblée lorsque ladite surface de raccordement de la première tige jouxte ladite surface de raccordement de la deuxième tige.

3. Le système de tige fémorale selon la revendication 2, ladite surface de raccordement (36) de la première tige étant substantiellement congrue à ladite surface de raccordement (34) de la deuxième tige.

4. Le système de tige fémorale selon la revendication 2, dans lequel :
ladite surface extérieure conique de la première tige (10) définit une première tangente (62) tracée à ladite surface de raccordement (36) de la première tige dans un plan frontal,
ladite surface extérieure conique de la deuxième tige (12) définit une deuxième tangente (64) tracée à ladite surface de raccordement (34) de la deuxième tige dans le plan frontal, et
ladite première tangente définissant un angle (θ) de moins de 10 degrés avec ladite deuxième tangente.

5. Le système de tige fémorale selon une quelconque des revendications 1 à 4, la première tige (10) étant dimensionnée et façonnée de façon à tenir dans une zone métaphysaire d'un fémur distal (F).

6. Le système de tige fémorale selon une quelconque des revendications 1 à 4, la deuxième tige (12) étant dimensionnée et façonnée pour tenir dans une zone diaphysaire d'un fémur distal (F) et une zone métaphysaire d'un tibia proximal.

7. Le système de tige fémorale selon une quelconque des revendications 1 à 4, au moins une des tiges que sont ladite première tige (10) et ladite deuxième tige (12) étant un biomatériau extrêmement poreux.
